# FASCICULE DE BREVET EUROPEEN

(11) **EP 0 737 463 B1**
(45) Date de publication et mention de la délivrance du brevet: **10.11.1999**
(21) Numéro de dépôt: 96400664.7
(22) Date de dépôt: 28.03.1996
(51) Int. Cl.: A61F 15/00, B65D 81/32

(54) **Emballage perdu compact formant un nécessaire**
Kompakte Einwegverpackungszusammenstellung
Compact disposable package kit

(30) Priorité: 10.04.1995 FR 9504243
(43) Date de publication de la demande: 16.10.1996
(73) Titulaire: Garconnet, Michel, F-76510 Saint-Nicolas-D'Aliermont (FR); Garconnet, Claudine Marie-Christine, 76510 Saint-Nicolas-D'Aliermont (FR); Garconnet, Armelle Annick, 76510 Saint-Nicolas-D'Aliermont (FR); Garconnet, Corinne Madeleine Gisèle, 76280 Criquetot L'Esneval (FR)
(72) Inventeur: Garç0nnet, Michel, 76510 St Nicolas d'Aliermont (FR)
(74) Mandataire: Coester, Jacques Charles

(56) Documents cités:
- DE-B- 1 087 758
- FR-A- 1 111 282
- FR-A- 1 202 856
- FR-A- 2 255 224
- US-A- 2 377 117

## Description

La présente invention concerne un nécessaire à utilisations multiples qui permet de conserver sous un faible encombrement et à l'abri de l'air plusieurs produits devant ensuite être utilisés ensemble ou successivement pour la réalisation d'actes divers.

Selon l'invention, des produits liquides, pâteux et/ou solides sont conditionnés dans un emballage et sont rendus utilisables à tous moments.

L'invention permet de faire en sorte que les mains d'un utilisateur puissent ne pas entrer en contact avec certains des produits, que ces produits soient toujours à l'abri de contaminations risquant d'en modifier les propriétés, que certains produits contenus soient amenés en contact les uns avec les autres avant ouverture du nécessaire tandis qu'ils demeurent isolés d'autres produits accessibles ultérieurement. L'invention permet, de plus, de faire en sorte que plusieurs nécessaires soient reliés entre eux pour leur vente et leur conservation tandis que leur utilisation n'intervient que successivement.

La présente invention trouve une application particulièrement appropriée pour la réalisation de pansements de première urgence disposés dans un emballage perdu et pouvant être utilisés en tous lieux tant par des personnes averties que par le grand public. Elle peut de même manière être appropriée au conditionnement de produits alimentaires, de produits cosmétiques, de produits de nettoyage, etc.

La technique antérieure a déjà fait connaître des étuis constituant des nécessaires pour des interventions sommaires.

FR-A-1 202 856 a fait connaître une enveloppe ou pochette qui contient un liquide par exemple un liquide désinfectant ainsi qu'un applicateur par exemple un tampon d'ouate la partie contenant le liquide étant séparée de la partie contenant le tampon d'ouate par un organe d'obturation pouvant être éjecté sous l'action d'une pression. L'étui comporte sur un côté une amorce de rupture permettant de le déchirer pour accéder au tampon qui peut être saisi avec les doigts.

Ce dispositif connu protège tant le liquide que le tampon tant qu'il n'est pas ouvert mais nécessite que le tampon, après son imprégnation soit saisi avec les doigts ce qui peut provoquer sa contamination par divers agents extérieurs.

US-A-2 377 117 décrit un étui qui se présente sous la forme d'un stylographe à l'intérieur duquel est placée une charge d'un liquide contenu dans un réservoir fermé par un embout poreux. Un rouleau d'un ruban autocollant est par ailleurs, disposé également à l'intérieur du corps de l'étui.

Dans cette réalisation, l'embout poreux peut évidemment être soumis à une contamination et il en est de même du ruban déroulable qui est destiné à former le pansement.

FR-A- 2 255 224 décrit un sachet d'emballage souple qui délimite une chambre de mélange et au mains une chambre réservoir qui peuvent être mises en communication par une ouverture d'une zone de rupture.

Cette publication ne prévoit pas d'éléments pouvant constituer un pansement de sorte que les produits pouvant être mélangés entre eux ne peuvent pas empêcher la contamination éventuelle d'un tampon en ouate, gaze ou autre matière devant être imprégnée.

DE-C-1 087 758 décrit un rouleau délimitant des segments successifs contenant respectivement des tampons poreux et des flacons contenant des liquides d'imprégnation. Dans ce cas également, au moins les tampons poreux peuvent être soumis à des contaminations.

FR-A-1 111 282 décrit un étui délimitant deux compartiments, l'un pour une ampoule contenant un liquide désinfectant et l'autre pour un pansement auto-adhésif. Lorsque l'étui est ouvert, tant l'ampoule que le pansement doivent être saisis avec les doigts, ce qui engendre un risque de contamination au moins du pansement.

L'invention a notamment pour objet un nouvel emballage formant un nécessaire pour pansement de première urgence dans lequel tant le tampon que le liquide d'imprégnation sont maintenus en permanence à l'abri de toute contamination, l'imprégnation du tampon étant ensuite réalisée sans contact manuel avec le tampon ni avec le liquide et l'utilisation du tampon imprégné pouvant s'effectuer également sans que les doigts de l'opérateur touchent le tampon imprégné de sorte que le tampon est à l'abri de toute contamination même si l'opérateur qui l'utilise n'a pas été à même de se laver les mains et de les désinfecter contrairement à ce que fait un praticien dans un cabinet médical.

En outre, l'invention permet, après le nettoyage d'une plaie par le tampon stérile imprégné d'un produit de traitement, de mettre en place un pansement protecteur sans que ce dernier ait de son côté été en contact avec les doigts de l'opérateur.

Ainsi, tant le nettoyage d'une blessure que la mise en place d'un pansement peuvent s'effectuer sans aucun risque de contamination.

Conformément à l'invention, l'emballage perdu compact pour nécessaire contenant des moyens de soins préservés de toute contamination, comporte une plaque de dos en matière plastique recouverte par une feuille formant un réservoir qui ouvre dans un petit conduit présentant une amorce de rupture, la feuille étant elle-même recouverte par un capot délimitant un logement pour un produit du genre gaze ou analogue, et la paroi supérieure du logement comportant un bouchon pour le maintien du produit et ayant des moyens pour en faciliter la séparation du capot ; la plaque, la feuille et le capot délimitant le logement étant assemblés et scellés de façon étanche et l'amorce de rupture étant disposée de façon que le petit conduit qui la comporte soit rompu dans le logement du capot lors d'un pliage de la plaque de dos pour amener un fluide contenu dans le réservoir à imprégner le produit disposé dans le logement du capot.

Suivant une autre caractéristique importante de l'invention, la plaque de dos est munie, sur sa face opposée à celle recouverte par la feuille, d'un sachet constitué par une pellicule autocollante elle-même recouverte par une feuille autocollante protégeant un moyen de recouvrement du genre pansement accessible par pelage de ladite feuille autocollante présentant un mode d'emploi.

Diverses autres caractéristiques de l'invention ressortent d'ailleurs de la description détaillée qui suit.

Une forme de réalisation de l'objet de l'invention est représentée, à titre d'exemple non limitatif, au dessin annexé.
La fig. 1 est une vue de dessus de l'emballage complet formant un nécessaire.
La fig. 2 est une vue de dessous du nécessaire de la fig. 1.
La fig. 3 est une vue latérale du nécessaire.
La fig. 4 est une vue latérale éclatée du nécessaire.

Dans ce qui suit, le nécessaire est décrit en tant qu'emballage pour pansement de première urgence sans être limité pour autant à cette réalisation.

Au dessin, une pellicule souple 1a d'un sachet 1 de forme générale rectangulaire est revêtue d'une couche de colle synthétique 2 sur au moins sa face tournée vers le haut.

La face encollée de la pellicule souple 1a est rendue adhérente d'une plaque de dos 3 dont la partie 3a représentée à droite du dessin fait face à un réservoir 4a contenant un produit actif qui est en général un fluide liquide plus ou moins visqueux.

De nombreux produits désinfectants peuvent convenir en tant que produit actif.

Le réservoir 4a est délimité par une feuille 4 en matière plastique rigide compatible avec celle constituant la plaque de dos 3 pour permettre leur liaison étanche par soudure ou un autre moyen.

Le réservoir 4a est prolongé par un petit conduit 5 formé comme lui par la partie supérieure de la plaque de dos 3 qui constitue le fond du réservoir.

Le petit conduit 5 présente, dans la partie de sa paroi formée par la feuille 4, une amorce de rupture 5a facilitant l'ouverture de ce conduit.

Un tampon 6 ou autre applicateur, constitué par de la gaze ou un autre produit pouvant être mis en charpie ou pouvant être plié en rond, en zigzag ou sous toute forme appropriée, est disposé sur le dessus de la plaque de dos 3.

Le tampon 6 de même que le réservoir 4a ainsi que le petit conduit 5 sont recouverts par un capot 8 en matière plastique transparente de même nature que celle constituant la feuille 4, ce capot 8 délimitant un cadre 8a recouvrant la périphérie de la feuille 4.

Le capot 8 délimite un logement 8₁ pour recouvrir le réservoir 4a et un logement 8₂ pour recouvrir le tampon 6.

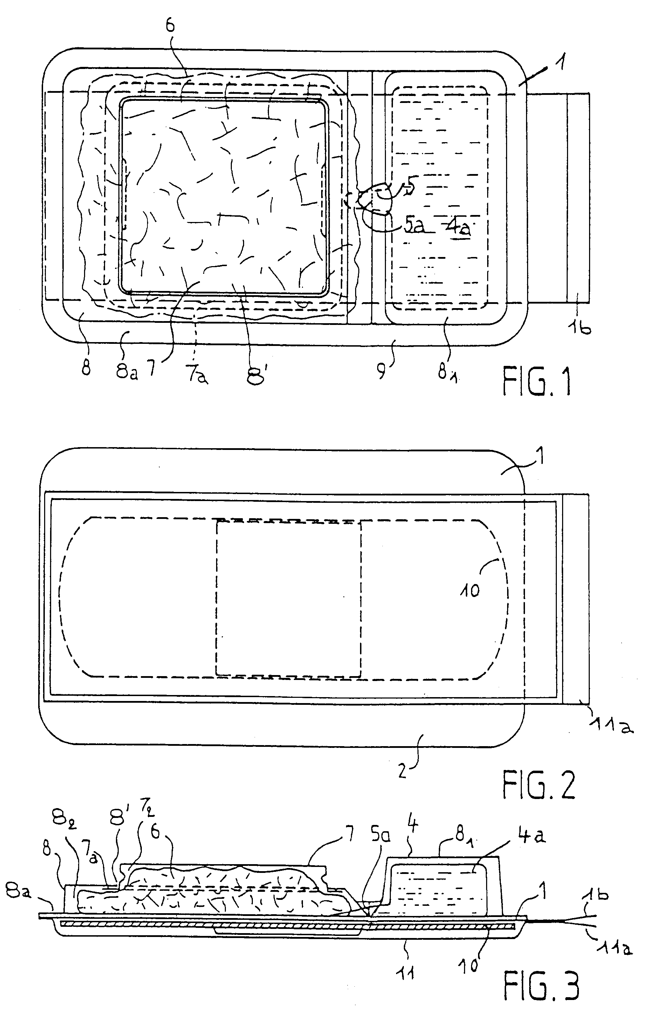

Comme le montre le dessin, il est avantageux que le logement 8₂ présente une ouverture 8' pour un bouchon 7 présentant un rebord 7a venant en appui contre le dessous de la partie supérieure du logement 8₂ ainsi que l'illustre la fig. 3.

Le bouchon 7 est creux et est rempli par le tampon 6 qui maintient le rebord 7a contre la paroi inférieure du logement 8₂ en assurant une bonne étanchéité entre ces deux organes.

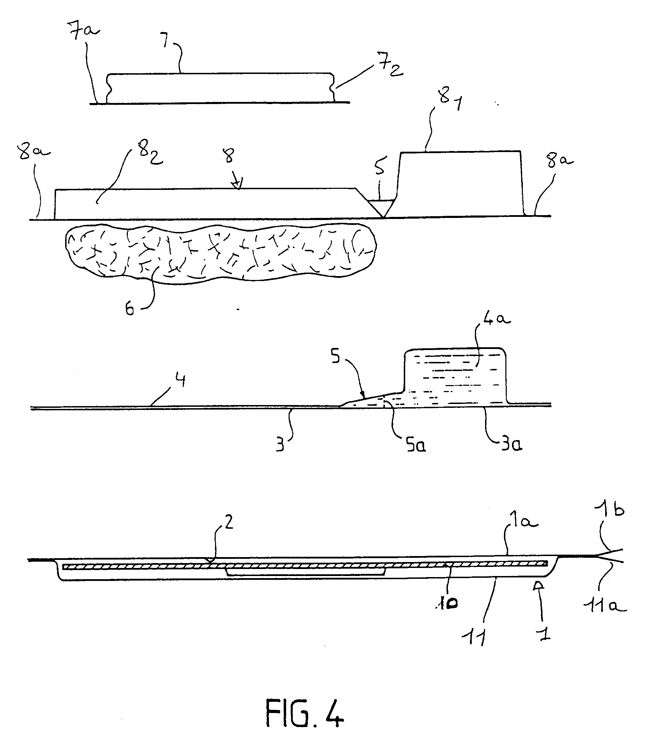

Ainsi que cela est illustré schématiquement aux fig. 3 et 4, le bouchon 7 présente éventuellement une paroi latérale 7₂ en forme de soufflet pour en permettre la déformation éventuelle.

La largeur du rebord 7a du bouchon 7 qui est réalisé en matière plastique souple analogue à celle du capot 8 est faible pour permettre une éventuelle extraction de ce bouchon comme cela est expliqué dans ce qui suit.

Il est possible également sans sortir du cadre de l'invention que le bouchon 7 soit formé d'une pièce avec la paroi du logement 8₂ et que le rebord 7a soit remplacé par une amorce de rupture analogue à l'amorce de rupture 5a.

Il est possible aussi, pour assurer une étanchéité totale entre la paroi du logement 8₂ et le bouchon 7, que le rebord 7a soit relié à la paroi du logement 8₂ par une matière d'étanchéification, par exemple une cire.

Le logement 8₁ qui recouvre le réservoir 4a est avantageusement mis à profit pour faire apparaître des signes graphiques imprimés sur la paroi interne de ce logement 8₁. Ces signes graphiques peuvent informer par exemple d'une référence de lot de fabrication, d'une date de péremption ou d'autres informations qui sont ainsi rendues indélébiles puisque non accessibles de l'extérieur.

Ainsi que cela est exposé dans ce qui précède, les différents organes décrits ci-dessus à l'exception du tampon 6 sont réalisés en matière plastique, cette matière étant de préférence, transparente au moins pour la réalisation du capot 8 afin que les signes graphiques ci-dessus soient clairement lisibles et que le tampon 6 ainsi que le produit contenu dans le réservoir 4a soient bien visibles.

La liaison entre le capot 8, la plaque de dos 3 et la feuille 4 est réalisée par collage ou par soudure ou un autre moyen approprié à la technique des matières utilisées pour la constitution de ces pièces.

Le sachet 1 est préparé séparément en disposant, de façon connue en soi, un pansement 10 ou un autre moyen de recouvrement contre la pellicule souple 1a et en recouvrant ce pansement et la pellicule par une feuille autocollante 11 dont la face inférieure visible comporte des informations concernant le mode d'emploi.

De préférence, tant la pellicule souple 1a que la feuille autocollante 11 de couverture du pansement 10 dépassent au-delà de la plaque de dos 3 comme montré en 1b et 11a au dessin.

Lorsqu'on désire utiliser le pansement, on brise le petit conduit 5 le long de l'amorce de rupture 5a en pliant la plaque de dos 3, la feuille 4 et le capot 8 entre les logements 8₁ et 8₂, ce qui fait que le produit actif liquide ou semi-liquide contenu dans le réservoir 4a imbibe le tampon 6. L'écoulement du produit actif est accéléré en pressant le réservoir 4a entre les doigts.

Lorsque le tampon 6 est imbibé, on extrait le bouchon 7 en le pressant entre les doigts, ce qui a pour effet de retirer en même temps le tampon 6 qui peut être utilisé pour nettoyer et désinfecter une plaie sans que le tampon ait été au contact des doigts et, par conséquent, sans que ce tampon ait pu subir une contamination quelconque puisqu'il demeure maintenu à l'intérieur du bouchon. Le maintien du tampon est encore amélioré lorsque le bouchon comporte un rebord 7₂ en forme de soufflet.

Une utilisation analogue du tampon maintenu dans le bouchon est obtenue lorsqu'une ligne de pré-rupture est prévue dans la paroi du logement 8₂.

Après nettoyage de la plaie, la feuille autocollante 11 du sachet 1 est pelée, ce qui permet l'application du pansement 10 sur la plaie sans que ce pansement ait été lui-même touché par les doigts de l'opérateur.

Cela évite également toute contamination du pansement.

Les extrémites 1b, 11a des feuilles constitutives du sachet 1 facilitent la manipulation de même que le collage de la pellicule souple 1a sur la plaque de dos 3 facilite la manipulation, cette plaque de dos constituant un support relativement rigide pour le sachet tant que la feuille 11 n'est pas pelée.

L'invention n'est pas limitée à l'exemple de réalisation, représenté et décrit en détail, car diverses modifications peuvent y être apportées sans sortir de son cadre tel que defini par les revendications annexées

## Revendications

1. Emballage perdu compact pour nécessaire contenant des moyens de soins préservés de toute contamination, comportant une plaque de dos en matière plastique (3) recouverte par une feuille (4) formant un réservoir (4a), caractérisé en ce que le réservoir (4a) ouvre dans un petit conduit (5) présentant une amorce de rupture (5a), la feuille (4) étant elle-même recouverte par un capot (8) délimitant un logement (8₂) pour un produit (6) du genre gaze ou analogue, et la paroi supérieure du logement (8₂) comportant un bouchon (7) pour le maintien du produit (6) et ayant des moyens pour en faciliter la séparation du capot (8) ; la plaque (3), la feuille (4) et le capot (8) délimitant le logement (8₂) étant assemblés et scellés de façon étanche et l'amorce de rupture (5a) étant disposée de façon que le petit conduit (5) qui la comporte soit rompu dans le logement (8₂) du capot (8) lors d'un pliage de la plaque de dos (3) pour amener un fluide contenu dans le réservoir (4a) à imprégner le produit (6) disposé dans le logement (8₂) du capot (8).

2. Emballage suivant la revendication 1, caractérisé en ce que le capot (8) délimite, supplémentairement, un second logement (8₁) recouvrant le réservoir (4a) délimité entre la feuille (4) et la plaque de dos (3).

3. Emballage suivant les revendications 1 et 2, caractérisé en ce que la paroi interne de la partie du capot (8) qui délimite le logement (8₁) comporte des signes graphiques.

4. Emballage suivant l'une des revendications 1 à 3, caractérisé en ce que la plaque de dos (3) est munie, sur sa face opposée à celle recouverte par la feuille (4), d'un sachet (1) constitué par une pellicule (1a) autocollante, elle-même recouverte par une feuille autocollante (11) protégeant un moyen de recouvrement du genre pansement (10) accessible par pelage de ladite feuille autocollante (11) présentant un mode d'emploi.

5. Emballage suivant l'une des revendications 1 à 4, caractérisé en ce que, tant la pellicule souple autocollante (1a) que la feuille autocollante (11) recouvrant le moyen (10), présentent des extrémités (1b, 11b) s'étendant au-delà du cadre (8a) que délimitent la plaque de dos (3), la feuille (4) et le capot (8) pour leur scellage au moyen de colle, soudure ou analogue.

6. Emballage suivant l'une des revendications 1 à 5, caractérisé en ce que le bouchon (7) est maintenu contre la paroi supérieure du logement (8₂) par un rebord (7a).

7. Emballage suivant l'une des revendications 1 à 6, caractérisé en ce que le bouchon (7) est formé par la paroi supérieure du capot (8) délimitant le logement (8₂) et en ce que l'amorce de rupture est formée autour dudit bouchon (7).

8. Emballage suivant l'une des revendications 1 à 7, caractérisé en ce que la paroi latérale du bouchon (7) est en forme de soufflet.

9. Emballage suivant l'une des revendications 1 à 8, caractérisé en ce qu'au moins le capot (8) est en matière plastique transparente.

## Claims

1. Compact disposable packing for kit containing aiding means which are secured from any contamination, comprising a back plate made of plastic material (3) covered with a sheet (4) that forms a reservoir (4a), characterized in that the reservoir (4a) opens in a small duct (5) having a breakage incipient area (5a), the sheet (4) being itself covered with a hood (8) that delimits a housing (8₂) for a product (6) like a gauze or similar, and the upper wall of the housing (8₂) comprising a plug (7) for maintaining the product (6) and having means for facilitating separation from the hood (8) ; the plate (3), the sheet (4) and the hood (8) that delimit the housing (8₂) being tightly assembled and sealed, and the breakage incipient area (5a) being arranged so that the small duct (5) that it comprises is broken in the housing (8₂) of the hood (8) upon bending the back plate (3) for causing a fluid contained in the reservoir (4a) to impregnate the product (6) arranged in the housing (8₂) of the hood (8).

2. Packing kit according to claim 1, characterized in that the hood (8) further delimits a second housing (8₁) covering the reservoir (4a) formed between the sheet (4) and the back plate (3).

3. Packing kit according to claims 1 and 2, characterized in that the inner wall of the portion of the hood (8) which delimits the housing (8₁) comprises graphical signs.

4. Packing kit according to one of claims 1 to 3, characterized in that the back plate (3) is provided, on its face opposed to that which is covered with the sheet (4), with a bag (1) which is formed by a self-adhesive film (1a), itself covered with a self-adhesive sheet (11) for protecting a covering means of the dressing type (10) which is accessible by peelling off said self-adhesive sheet (11) that is provided with directions of use.

5. Packing kit according to one of claims 1 to 4, characterized in that that both the self-adhesive flexible film (1a) and the self-adhesive sheet (11) that covers the means (10), are provided with ends (1b, 11b) extending beyond the frame (8a) delimited by the back plate (3), the sheet (4) and the hood (8) for a sealing thereof by means of a glue, a solder or similar.

6. Packing kit according to one of claims 1 to 5, characterized in that the plug (7) is maintained on the upper wall of the housing (8₂) by an edge (7a).

7. Packing kit according to one of claims 1 to 6, characterized in that the plug (7) is formed by the upper wall of the hood (8) that delimits the housing (8₂), and in that the breakage incipient area is formed around said plug (7).

8. Packing kit according to one of claims 1 to 7, characterized in that the side wall of the plug (7) is bellows shaped.

9. Packing kit according to one of claims 1 to 8, characterized in that at least the hood (8) is made of a clear plastic material.

## Patentansprüche

1. Kompakte Einwegverpackung für ein vor jeder Verseuchung geschüzte Pflegemittel enthaltendes Nutzzeug, mit einer mit einer einen Behälter (4a) bildenden Folie (4) überdeckten Rückenplatte aus Kunststoff (3), dadurch gekennzeichnet, daß der Behälter (4a) in einen kleinen, einen Bruchquellenanriß (5a) aufweisenden Kanal (5) ausmündet, wobei die Folie (4) selber durch eine eine Aufnahme (8₂) für ein Erzeugnis (6) der Gazeart oder dergleichen abgrenzende Haube (8) überdeckt ist und wobei die obere Wand der Aufnahme (8₂) einen Abschlussdeckel (7)für das Halten des Erzeugnisses (6) aufweist und Mittel zur Erleichterung der Abtrennung der Haube (8) hat ; wobei die die Aufnahme (8₂) abgrenzende Platte (3), die Folie (4) und die Haube (8) zusammengefügt und dicht verschlossen sind und wobei der Bruchquellenanriß (5a) derart angeordnet ist, daß der Kleine Kanal (5), der ihn aufweist, innerhalb der Aufnahme (8₂) der Haube (8) während eines Umknickens der Rückenplatte (3) gebrochen wird, um ein in dem Behälter (4a) enthaltenes Fließmittel zu veranlassen, das in der Aufnahme (8₂) der Haube (8) liegende Erzeugnis (6) zu tränken.

2. Verpackung gemäß Anspruch 1, dadurch gekennzeichnet, daß die Haube (8) zusätzlich eine den zwischen der Folie (4) und der Rückenplatte (3) abgegrenzten Behälter (4a) überdeckende zweite Aufnahme (8₁) abgrenzt.

3. Verpackung gemäß den Ansprüchen 1 und 2, dadurch gekennzeichnet, daß die Innenwand desjenigen Teiles der Haube (8), der die Aufnahme (8₁) abgrenzt, graphische Zeichen aufweist.

4. Verpackung nach einem der Ansprüche 1 bis 3, dadurch gekennzeichnet, daß die Rückenplatte (3) auf ihrer der durch die Folie (4) überdeckten Seite entgegengesetzten Seite mit einem Beutel (1) versehen ist, der durch eine selbstklebende Haut (1a) gebildet wird, die mit einer selbstklebenden Folie (11) überzogen ist, welche ein Abdeckmittel der Verbandart (10) schützt, das durch abschälen der besagten eine Gebrauchsanweisung aufweisenden selbstklebenden Folie (11) zugänglich ist.

5. Verpackung gemäß einem der Ansprüche 1 bis 4, dadurch gekennzeichnet, daß sowohl die nachgiebige selbstklebende Haut (1a) als auch die das Mittel (10) abdeckende selbstklebende Folie (11) Enden (1b, 11b) aufweisen, die sich über den Rahmen (8a), den die Rückenplatte (3), die Folie (4) und die Haube (8) abgrenzen, hinaus erstreckt, für deren abdichtenen Verschluß mittels Klebstoff, Schweißen oder dergleichen.

6. Verpackung gemäß einem der Ansprüche 1 bis 5, dadurch gekennzeichnet, daß der Abschlußdeckel (7) an der oberen Wand (8₂) durch eine Randleiste (7a) gehalten wird.

7. Verpackung nach einem der Ansprüche 1 bis 6, dadurch gekennzeichnet, daß der Abschlußdeckel (7) durch die obere Wand der die Aufnahme (8₂) abgrenzenden Haube (8) gebildet wird und daß der Bruchquellenanriß um den besagten Abschlußdeckel (7) herum gebildet wird.

8. Verpackung gemäß einem der Ansprüche 1 bis 7, dadurch gekennzeichnet, daß die Seitenwand des Abschlußdeckels (7) die Gestalt eines Balges hat.

9. Verpackung gemäß einem der Ansprüche 1 bis 8, dadurch gekennzeichnet, daß wenistens die Haube (8) aus durchsichtigem Kunstoff ist.
